# EUROPEAN PATENT SPECIFICATION

(11) **EP 2 934 330 B1**
(45) Date of publication and mention of the grant of the patent: **11.10.2017**
(21) Application number: 13864454.7
(22) Date of filing: 19.12.2013
(51) Int. Cl.: A61B 8/12, A61F 2/01

(54) **IMPLANT DELIVERY SYSTEM AND IMPLANTS**
IMPLANTATEINFÜHRUNGSSYSTEM UND IMPLANTATE
SYSTÈME DE POSE D'IMPLANT, ET IMPLANTS

(30) Priority: 20.12.2012 US 201261740196 P; 12.03.2013 US 201361777619 P
(43) Date of publication of application: 28.10.2015
(73) Proprietor: Stigall, Jeremy, San Diego, CA 92130 (US); Lauinger, Joseph, San Diego, CA 92130 (US); Goodman, David, San Diego, CA 92130 (US)
(72) Inventor: Stigall, Jeremy, San Diego, CA 92130 (US); Lauinger, Joseph, San Diego, CA 92130 (US); Goodman, David, San Diego, CA 92130 (US)
(74) Representative: de Haan, Poul Erik
(86) International application number: PCT/US2013/076480
(87) International publication number: WO 2014/100382

(56) References cited:
- WO-A2-2012/003369
- WO-A2-2012/003369
- US-A1- 2008 027 481
- US-A1- 2009 203 962
- US-A1- 2009 318 951
- US-A1- 2012 041 533

## Description

### Technical Field

The present invention generally relates to an apparatus for guided intraluminal placement of implants, such as vena cava filters, into the vasculature.

### Background of the Invention

Deep vein thrombosis is a medical condition that results from the formation of a blood clot, or thrombus, within a vein. Thrombi often develop in the calves, legs, or other lower abdomen, but may occur in other vessels. The clot is typically formed from a pooling of blood within the vein due to abnormally long periods of rest, e.g. when an individual is bed ridden following surgery or suffering a debilitating illness. Other causes of thrombosis include genetic deficiencies, autoimmune disorders, and endothelial cell injury.

The thrombus may partially or completely block blood flow. In some circumstances, the thrombus may break off and travel through the blood stream causing serious health issues. For example, when a thrombus of the lower extremities breaks off, the thrombus may travel through the lungs and cause a pulmonary embolism. A pulmonary embolism is a blockage of the blood supply to the lungs that causes severe hypoxia and cardiac failure. It frequently results in death.

Thrombi may be treated with anti-coagulant drugs, such as heparin treatment, to dissipate the thrombus and prevent the thrombus breaking off into the bloodstream. However, such therapy is often ineffective for preventing recurrent thrombi or is not suitable for use in patient with acute sensitivity to heparin. The current standard of treatment, as an alternative to anti-coagulant drug therapies, is the intravascular insertion and implantation of a vena cava filter. Typically, vena cava filters are expandable wire cage-like devices that include a plurality of wire legs with hook ends. When the vena cava filter is deployed into the vena cava, the wire legs expand radially and the hook ends secure onto the luminal wall of the vena cava.

In order to place a vena cava filter into the vasculature, most current practices require a fluoroscopy unit to determine the proper implantation location for the filter with respect to the thrombus. However, the fluoroscopy techniques are expensive, not suitable for overweight patients, and may expose the patient to potentially nephrotoxic contrast media.

As alternative and in addition to fluoroscopy procedures, some intraluminal imaging technologies have emerged to guide vena cava filter implantation. For example, U.S. Patent No. 6,645,152 describes an intraluminal device that includes an imaging catheter connected in parallel to a separate delivery catheter. The imaging catheter extends distal and next to the opening of the delivery catheter in order to image a filter being deployed out of the opening. As the filter expands after deployment, the filter legs must maneuver around imaging catheter to engage with the wall of the vena cava. This can interfere with image quality, damage the imaging catheter and/or result in filter misplacement. In addition, U.S. Patent No. 6,440,077 describes an imaging catheter disposed within a delivery catheter and configured to pass through a filter to imaging the vessel prior to and during implantation. This device requires a specially designed filter that includes an opening large enough for the imaging catheter to pass through. Moreover, this device also risks filter misplacement caused by forward and backward movement of the imaging catheter through the implanted filter.

US2012041533 A1 discloses various devices that are described for imaging a body lumen during delivery and deployment of a medical device. In one example, a delivery device includes at least one sheath, a stent, an inner tubular member and at least one imaging device to allow visualization of the stent prior, during and after deployment without the use of an endoscope. The inner tubular member includes an articulating portion with the imaging device integrally formed and embedded therein.

### Summary

The invention recognizes that current intraluminal imaging devices do not provide for real-time imaging of the vessel area during the filter delivery procedure without interfering with the filter during implantation and/or as implanted. In addition, the invention recognizes the need for an intraluminal and delivery device with a smaller profile (e.g. does not merely connect two separate catheters in parallel) and that is compatible with commercially available filters (e.g. does not require a specially designed filter with an opening large enough to fit an imaging catheter). Insofar as the term "invention" and or "embodiment" are used in the following, and/or features are presented as being optional, this should be interpreted in such a way that the only protection sought is that of the invention as claimed. Aspects of the invention are accomplished by providing an implant delivery device as disclosed in the appended claims that is configured to releasably hold an implant within a center lumen and includes a first imaging element configured to at least partially surround the center lumen such that the implant is deployable through the imaging element. For deployment of the implant, the implant delivery device of the invention includes an inner member moveably disposed within the center lumen of the implant delivery device. The inner member can be used to push the implant so that the implant deploys out of the center lumen. The first imaging element does interfere with implant deployment because the implant passes through the first imaging element and then deploys into the body lumen. Moreover, this configuration provides a smaller profile because the implant and first imaging element are concentrically aligned. The inner member includes a second imaging element. After the implant is deployed into a vessel and engaged with a vessel wall, the inner member may enter the vessel and the second imaging element may be used to image the implant as positioned within the vessel. A particular benefit of this aspect is that the second imaging element can image the implant as engaged with the vessel wall without touching or interfering with the implant because the inner member has a smaller profile than the elongate body and is able to fit within a cavity formed by the deployed implant. For example, if the implant is filter, the filter legs expand from a center point and engage with the vessel walls, thereby creating a funnel-like cavity between the filter legs. The inner member may move within the funnel-like cavity between the expanded wire legs to image the legs as engaged with the vessel wall.

Devices of the present invention may be used in a variety of body lumens, including but not limited to intravascular lumens such as coronary arteries. Typically, devices of the invention are used to implant filters within the vasculature to prevent thrombi from travelling within the bloodstream. However, devices of the invention can be used to delivery other implants for a variety of reasons. For example, the implant may be introduced into a vessel to occlude the vessel downstream and eliminate blood flow within the vessel. In another example, the implant may be introduced into a vessel to provide open mechanical support to a diseased vessel. Suitable implants for delivery into a body lumen include, but are not limited to a plug, a stent, a pH sensor, a pressure monitor, a plug, a filter, or a valve. The implant may be expandable, such as self-expandable stents or filters.

In certain aspect, the implant delivery device of the invention includes an elongate body and an inner member. The elongate body includes a first imaging element and defines a center lumen that leads to an opening. The elongate body is configured to releasably hold an implant within the center lumen. The first imaging element is configured to at least partially surround the center lumen such that the implant is deployable through the first imaging element. The inner member is moveably disposable within the center lumen of the elongate body. The inner member is configured to engage with and deploy the implant out of the opening and into a body lumen.

The first imaging element may be positioned on or formed as part of an outer surface of a distal end of the elongate body. In certain embodiments, the first imaging element surrounds the distal end of the elongate body at a position slightly proximal (e.g. two millimeters or less) to the opening of center lumen. With this arrangement, the operator is able to locate an implantation site with the first imaging element, and then position elongate body for implant deployment such that the opening is slightly proximal to the located implantation site. In this manner, the operator can know that the implant is being delivered at a location slightly distal to real-time images being obtained from the first imaging element.

In order to facilitate deployment of the implant out of the center lumen and into a body lumen, the inner member includes a push element configured to engage with the implant. For example, the push element may include a substantially flat surface that is flush with the wall of the center lumen. The push element may form the distal end of the inner member. The inner member further includes a second imaging element. The second imaging element is proximal to the push element of the inner member. The second imaging element can be used to obtain images of the luminal surface when at least a portion of the inner member is deployed out of the opening of the center lumen. In this manner, the second imaging element allows one to obtain images of the implant as implanted within the body lumen. The present disclosure further includes methods for delivery an implant into a body lumen. According to some examples, the method includes introducing an implant delivery device into a body lumen. The implant delivery device includes an elongate body with a first imaging element and a center lumen that leads to an opening. The first imaging element is configured to at least partially surround the center lumen. The device further includes an implant releasably held within the center lumen and an inner member being moveably disposed within the center lumen. The method further includes imaging a surface of the first imaging element to locate an implantation site, positioning the elongate body for deployment of the implant based on the imaging step, and deploying the implant out of the opening and into the implantation site.

The first and second imaging elements can be a component of any known intraluminal imaging apparatus. Suitable imaging apparatus for use with the implant delivery device of the invention include, for example, optical-acoustic sensor apparatuses, intravascular ultrasound (IVUS) or optical coherence tomography (OCT).

Other and further aspects and features of the invention will be evidence from the following detailed description and accompanying drawings, which are intended to illustrate, not limit, the invention.

### Brief Description of the Drawings

FIG. 1 depicts a distal end of an implant delivery device according to one embodiment.
FIG. 2 shows a connector fitting that connects to devices of the invention.
FIG. 3 depicts a longitudinal cross-section of a distal portion of the delivery device according to certain embodiments.
FIG. 4 depicts another cross-section along the x-axis of a distal portion of the delivery device according to certain embodiments.
FIG. 5 depicts an alternative embodiment of the delivery device.
FIGS. 6-11 illustrate an exemplary delivery device of the invention in operation.
FIG. 12A-12C depicts filters according to certain embodiments.
FIG. 13 is a system diagram according to certain embodiments.
FIGS. 14A-B depict an alternative embodiment of the delivery device.

### Detailed Description

The present invention generally relates to delivery systems with combined intraluminal implant delivery and imaging capabilities. The delivery systems of the invention provide for 1) real-time imaging of intraluminal surfaces to detect a location of interest (e.g. implantation site); 2) delivery of an implant into the implantation site; and 2) real-time imaging of the implant as engaged with the intraluminal surface without interfering with the implant as implanted. Because the above features are accomplished with one system introduced into a body lumen, this present invention eliminates the need to introduce multiple catheters into the body. For example, there is no need to introduce and remove an imaging catheter to locate a region of interest, then introduce and remove a delivery catheter to deliver an implant, and then re-introduce the imaging catheter to evaluate the implant as implanted.

The delivery system may be used to deliver any suitable implant into a body lumen. Suitable implants for delivery into the lumen include a stent, a plug, a pH sensor, pressure monitor, a plug, a filter, or a valve. The delivery system may be used to deliver an implant for a variety of reasons. For example, the implant may be introduced into a vessel to occlude the vessel downstream to eliminate blood flow within the vessel. In another example, the implant may be introduced into a vessel to provide open mechanical support to a diseased vessel. The implant used for purposes of describing the components and function of the delivery system is a filter.

In certain embodiments, systems of the invention image and deliver an implant within a lumen of tissue. Various lumen of biological structures may be imaged and receive an implant including, but not limited to, blood vessels, vasculature of the lymphatic and nervous systems, various structures of the gastrointestinal tract including lumen of the small intestine, large intestine, stomach, esophagus, colon, pancreatic duct, bile duct, hepatic duct, lumen of the reproductive tract including the vas deferens, uterus and fallopian tubes, structures of the urinary tract including urinary collecting ducts, renal tubules, ureter, and bladder, and structures of the head and neck and pulmonary system including sinuses, parotid, trachea, bronchi, and lungs.

In particular embodiments and discussed hereinafter, the delivery system of the invention is used to deploy a filter into vessel of the vasculature to block thrombi from traveling through the blood stream. These types of filters are typically introduced into the inferior vena cava vein to block thrombi originating in the lower extremities from breaking off and traveling through the bloodstream. Prior to filter placement, the surgeon must take care to locate the proper filter location. The optimal location for filter placement is in the infrarenal inferior vena cava with the apex of the filter just below the level of the lowest renal vein because, at this level, a thrombus caught by the filter will be exposed to renal vein blood flow, which may promote dissolution by the intrinsic lytic system. In order to determine appropriate placement of a vena cava filter, a surgeon must take care to avoid encroachment on the renal veins and to ascertain the absence or presence of thrombus within the vena cava. For example, a filter placed at or above the renal veins can lead to renal vein thrombosis and deterioration of renal function if the filter, and thus the vessel, become occluded. In addition, the filter should not be placed on a thrombus tissue, but rather should be placed on healthy vessel walls to ensure the filter engages with the wall of the vena cava.

The delivery system of the invention may optionally involve the introduction of an introducer sheath. Introducer sheaths are known in the art. Introducer sheaths are advanced over the guidewire into the vessel. A catheter or other device may then be advanced through a lumen of the introducer sheath and over the guidewire into a position for performing a medical procedure. Thus, the introducer sheath may facilitate introducing the catheter into the vessel, while minimizing trauma to the vessel wall and/or minimizing blood loss during a procedure.

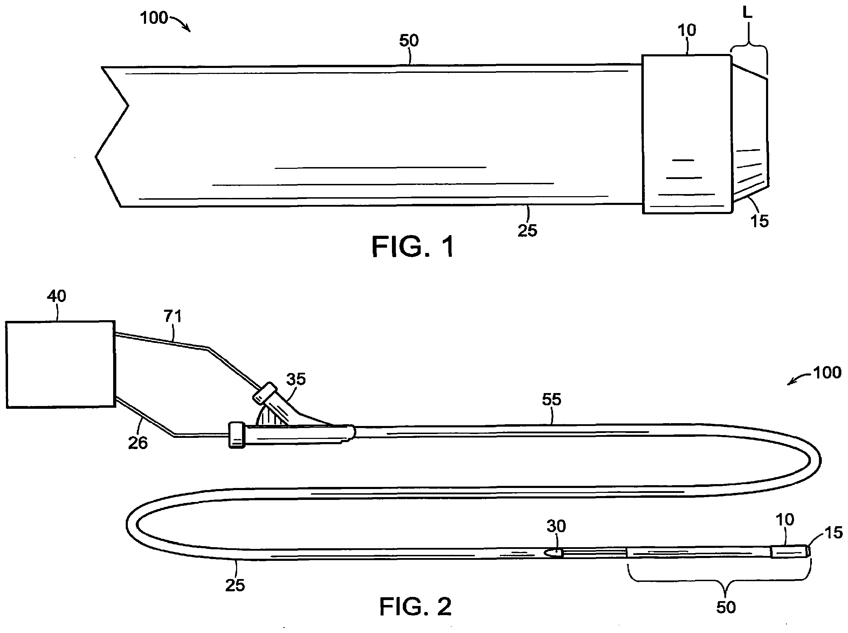

FIG. 1 depicts the distal portion 50 of a delivery system 100 according to certain embodiments. The delivery system 100 includes an elongate body 25 and an imaging element 10 located near a distal tip 15 of the elongate body 25. An opening (not shown in FIG. 1) is open in the distal direction on the distal tip 15, through which an implant can be delivered into a body lumen. The imaging element 10 is a distance L from the distal tip 15. In certain embodiments, the imaging element is positioned 2 millimeters or less from the distal tip 15. Ideally, the imaging element 10 is positioned as close as possible to the distal tip 15 to minimize the distance between the actual location of the imaging and an implant delivery site. The imaging element 10 can partially or fully surround the elongate body 25. In addition, the imaging element 10 can be positioned on or formed as part of an outer surface of the distal end 50 of the elongate body 25. In preferred embodiments, the imaging element 10 surrounds the elongate body 25 to provide cross-sectional imaging of the body lumen (i.e. to provide a 360 degree slice of the vessel at different longitudinal sections of the body lumen). If the imaging element 10 only partially surrounds the elongate body 25, the elongate body 25 could be configured to rotate to provide cross-sectional imaging.

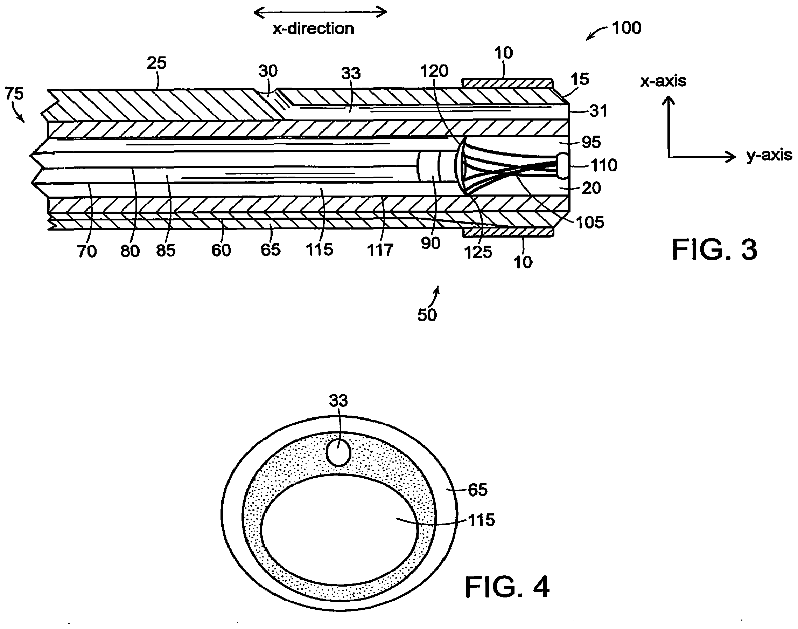

FIG. 2 shows a connector fitting that connects to devices of the invention. Connector fitting 35 is attached at a proximal portion 55 of the elongate body 25. Connector fitting 35 provides a functional access port at the proximal end of devices of the invention. For example, the inner member 70 (not shown in FIG. 2) can be telescoped through the elongate body 25 through the connector fitting 35. Through the connector fitting 35 and via cable 26, the elongate body 25 can be operably coupled to an operating system 40. In addition, a proximal end 71 of the inner member 70 (shown within the lumen of the elongate body in FIG. 3) can be coupled to the operating system 40. Typically, the operating system 40 provides a means to transmit electricity and receive imaging data from imaging elements of the delivery systems. The operating system 40 can be a component of computerized ultrasound assembly equipment, optical coherence tomography assembly equipment, or equipment of another imaging system. Various imaging assemblies suitable for use with devices of the invention are described in more detail hereinafter. In addition, the operating system 40 can be configured to provide a controlled translation of the inner member with respect to the elongate body 25. As alternative to one operating system, the elongate body 25 and the inner member 70 can be coupled to separate operating systems.

FIG. 2 also shows a guidewire opening 30 near the distal portion 50 of the elongate body 25. As shown in FIG. 2, the delivery system 100 is a rapid exchange catheter device. However, the delivery system 100 can be designed as an over-the-wire system or a rapid exchange system. Over-the-wire catheters include a guidewire lumen that runs the full length of the catheter. Rapid exchange catheters include a guidewire lumen extending only through a distal portion of the catheter. With respect to the remaining proximal portion of the catheter, the guidewire exits the internal catheter lumen through a guidewire opening 30, and the guidewire extends in parallel along the proximal catheter portion.

FIG. 3 depicts a cross-section of the distal portion 50 of the delivery device 100 according to certain embodiments. The delivery device 100 includes an elongate body 25 that defines a center lumen 115. The center lumen 115 extends to an opening 20 through which an implant can be deployed. The elongate body 25 includes an imaging element 10 positioned on or formed as part of the outer surface. The imaging element 10 is a component of an imaging assembly, which are described in more detail hereinafter. The imaging element 10 of the elongate body is connected to transmission line 60. The transmission line 60 transmits electricity to the imaging element 10 and receives imaging signals from the imaging element 10. The transmission line 60 is disposed through a transmission lumen 65 of the elongate body. The transmission line 60 can include one or more signal lines.

An inner member 70 is disposed within the center lumen 115 of elongate body 25. The inner member 70 is movable within the center lumen 115 and can translate with respect to the elongate body 25 in the forward (distal) and backward (proximal) directions, as indicated by arrow x. In addition, the elongate body 25 can translate and move relative to the inner member 70. In certain embodiments, the inner member 70 is moved distally within the center lumen 115 to engage the inner member 70 with a filter 95 and to push the filter 95 into a body lumen.

The inner member 70 includes a push member 120 located at a distal end of the inner member 70. The push member 120 engages with a proximal end of the filter 95. As shown in FIG. 3, the push member 120 engages with the hooked ends 125 of the filter legs 105. The push member 120 can be a flat or slightly-cupped shaped surface (as shown) and can extend the width of the center lumen. The slightly-cupped shaped surface of the push member 120 acts to contain the legs and minimize the hooks ends 125 from engaging with the surface 117 of the center lumen 115. In addition, the push-member can be shaped to specially mate with the filter being deployed. In certain embodiments, the surface 117 of the center lumen 115 that is exposed to the filter hooks ends 125 is formed of a material that prevents the hook ends 125 from perforating, penetrating, or catching on the surface 117 of the center lumen 115 during deployment of the filter 95.

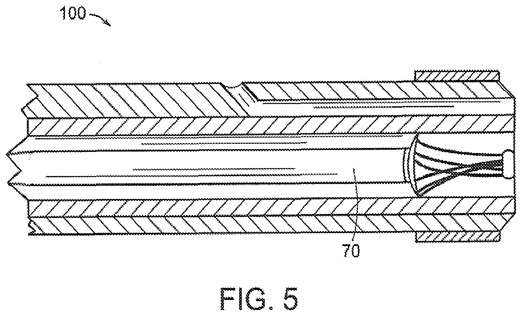

In certain embodiments and as shown, the inner member 70 includes a center member imaging element 90. The center member imaging element 90 is a component of an imaging assembly, which are described in more detail hereinafter. The center member imaging element 90 is proximal to the push member 120. Preferably, the distance between the center member imaging element 90 and the push member 120 is minimized so that the imaging element 90 substantially images from a distal end of the inner member 70. Like the imaging element 10 of the elongate body 25, the imaging element 90 of the inner member 70 can surround the inner member to provide for cross-sectional imaging (360 degree) of the body lumen. If the center member imaging element 90 only partially surrounds the inner member 70, the inner member 70 could be configured to rotate to provide cross-sectional imaging. The center member imaging element 90 is connected to a transmission line 80, which transmits electricity to the center member imaging element 90 and receives imaging signals from the imaging element 90. The transmission line 80 can include one or more signal lines. The transmission line 80 can be disposed within a lumen 85 of the inner member 70. Alternatively, the transmission line 80 can be integrated into the body of the inner member 70. FIG. 5 depicts an alternative embodiment of the delivery device in which the inner member 70 does not include a center member imaging element.

The elongate body 25 further includes a rapid exchange guidewire lumen 33. The guidewire lumen has a guidewire opening 30 at a proximal end and a guidewire opening 33 at a distal end. The elongate body 25 can be guided over a guidewire (not shown) extending through the guidewire lumen 33. During implant deployment, the guidewire can be retracted into the guidewire lumen 33 to prevent the guidewire from interfering with the implant 95 as it is being deployment. Alternatively, the delivery device 100 can be configured as an over-the-wire device.

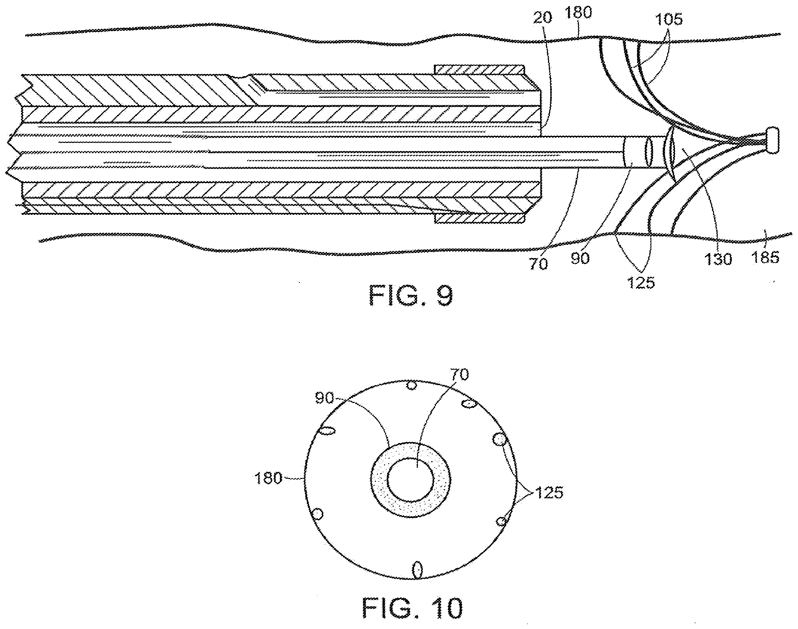

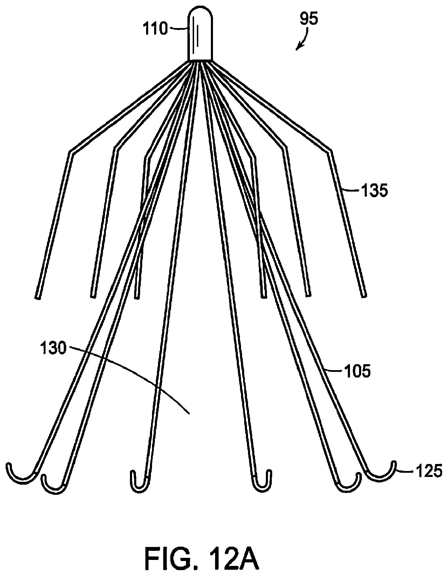

The elongate body 25 is also configured to releasably hold an implant, such as the filter 95 as shown. The filter 95 includes a plurality of legs 105 connected to a center hub 110. The filter legs 105 are configured to expand radially to engage with a surface of a body lumen when fully deployed. As shown in FIG. 3, the filter legs 105 are in a contracted state. FIGS. 9 and 12a depict a filter 95 in its fully expanded state. The filter 95 includes a plurality of legs 150 with hook ends 125. The hook ends 125 secure the filter 95 into the body lumen. The plurality of legs 105 forms a funnel-like cavity 130 between the legs 105. As discussed more fully hereinafter, the inner member 70 can move within the funnel-like cavity 130 of an implanted filter 95 to obtain images of the filter as implanted. In addition and as shown in FIG. 12A, a filter 95 can include capture members 135 that act to further prevent a thrombus from passing through the filter. In addition to the filter 95 shown, most commercially available filters can be used with the delivery device of the invention. Suitable filters are described in U.S. Patent Nos. 6468290, 7534251, and 7972353.

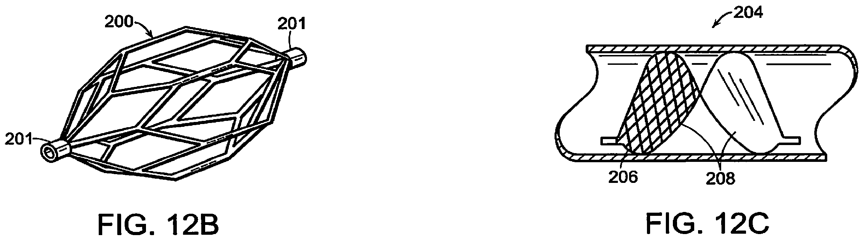

FIGS. 12B-12C illustrate other filters suitable for use in devices of the invention. FIG. 12B illustrates a caged filter 200 that includes a mesh, collapsible cage body disposed between end portions 201. The end portions 201 define a lumen so that the cage filter may be ridden over a guidewire. FIG. 12C depicts a winged-filter 208 disposed within a vessel. The winged-filter 209 includes an expandable two-part frame with a mesh portion 206 disposed within at least one part of the frame.

Each of the filters illustrated in FIGS. 12A-12C are configured to expand to securely-fit against the vessel wall. This allows the filters to capture/snare blood clots traveling through the vasculature without risk of dislocating the filters.

Typically, a filter implanted within a blood vessel is retrieved from the vessel after a certain period of time. There are often several issues associated with the retrieval that can lead to damage to the patient's vessel or result in leaving the filter within the patient permanently, which could deteriorate the vessel wall. In order to overcome the issues associated with retrieval of filters and filters left in the body permanently, certain embodiments of the invention provide filters with bioabsorable properties that decay over time and eventually absorb into the body's blood stream and/or tissue. A filter of the invention may be made with any material having bioabsorable properties, such as magnesium alloys and bioabsorbable polymers. Specifically, bioabsorbable material may include, for example, magnesium alloys, polyglycolic acid, polygalctin 910, poliglecaprone, polydioxanone, poly-α-hydroxy acids, e.g. polylactides, polyglycolides and their copolymers, polyanhydrides, polyorthoesters, segmented block copolymers of polyethylene glycol and poly terephthalate, tyrosine derivative polymers or poly(ester amides). Filters having one or more components may be formed from the same or different bioabsorable materials.

In certain embodiments, the elongate body can include radiopaque markers on the distal tip 15 and the imaging element 10 to assist in determining the location of the elongate body in the vasculature relative to the images obtained by the imaging element. This will allow an operator to visualize the location of the delivery device within the vasculature via an angiogram. The imaging obtained by imaging element 10 may be co-located with the radiopaque markers as described in co-assigned and co-pending application entitled, "LOCATING INTRAVASCULAR IMAGES".

FIG. 4 depicts a cross-section along the x-axis of the elongate body 25 shown in FIG. 3. FIG. 4 illustrates the lumens of elongate body 25. As discussed, the elongate body 25 includes center lumen 115, transmission lumen 65, and a guidewire lumen 33. The transmission lumen 65 does not have to fully surround a portion of the elongate body 25.

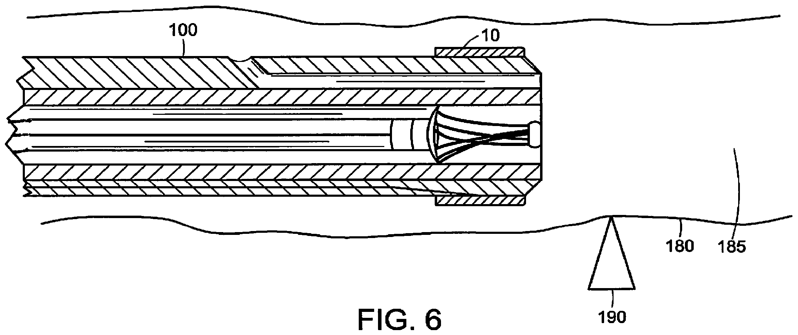

FIGS. 6-11 illustrate the delivery device illustrated in FIG. 3 in operation. FIG. 6 shows the delivery device 100 disposed within a lumen 185 of vessel 180. The delivery device can be introduced in to a vessel using methods known in the art. Typically, a guidewire is inserted into the vessel using the Seldinger technique and the delivery device is guided over the guidewire to the vessel and region of interest. Once the delivery device 100 is inserted, the operator can obtain real-time images of the luminal surface of the vessel using imaging element. Using the real-time imaging of the luminal surface of the vessel 185, the operator is able to locate a target implantation site, such as target implantation site 190.

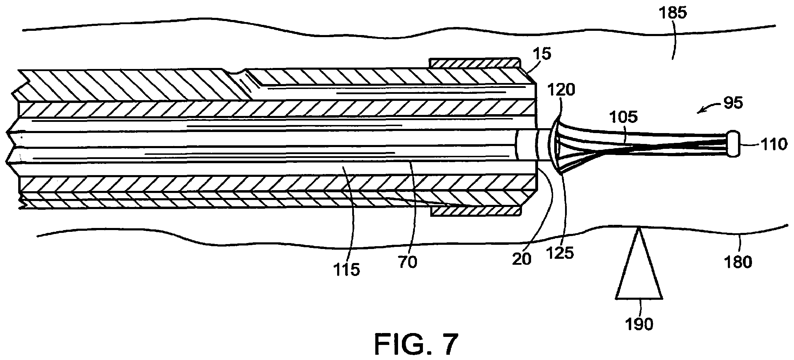

After the target implantation site 190 is located, the operator places the distal tip 15 of the delivery device proximal to the target implantation site 190 (as shown in FIG. 7). A user interface module (included in operating systems 40a and 40b) shown in FIG. 1) connected to the imaging element 10 and the elongate body 25 can assist the operator in determining the amount of pull back of the elongate body 25 required so that the distal tip 15 is located proximal to the implantation site 190 located by the imaging element 10.

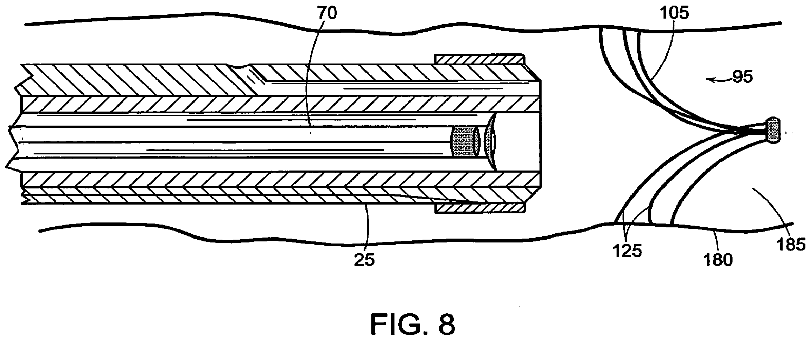

Once the elongate body 25 is positioned for deployment, the inner member 70 is moved distally through the center lumen 115 of the elongate body 25 to push the filter 95 out of the opening 20 of the distal tip 15. As shown, the push member 120 of the inner member 70 engages with the filter legs 105 to deploy the filter 95 into the vessel lumen 185 towards the implantation site 190. Once the proximal ends 125 of the filter legs 105 exit into the vessel lumen 185 from the opening 20, the legs 105 spring open and attach themselves via the hook ends to the vessel wall 180. To assist with expansion of the filter legs 105, the inner member 70 can be retracted back into the center lumen 115 and away from the filter legs 105. FIG. 8 shows the filter 95 as implanted within the vessel 180 with the inner member 80 retracted back into the elongate body 25.

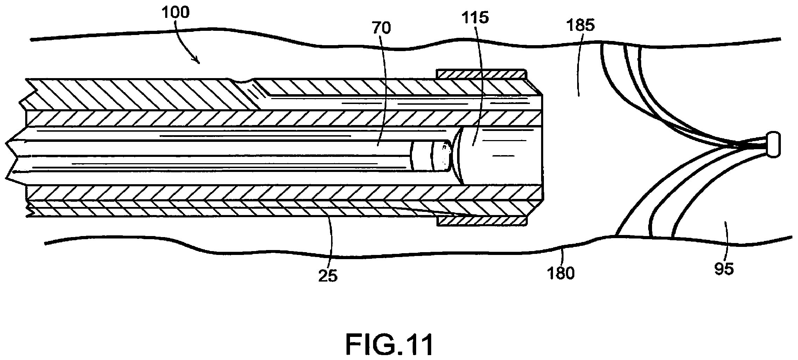

When the filter is placed in the vessel, the inner member 70 can be deployed out of the opening 20 and into the vessel lumen 185 to provide real-time images of the filter 95 as engaged with the vessel wall 180. As shown in FIG. 9, the inner member 70 is deployed into the opening and into the funnel-like cavity 30 formed between the plurality of filter legs 95. The imaging element 90 of the inner member 70 can obtain real-time images to evaluate and ensure that the filter leg hook ends 125 of the filter 95 are properly attached to the wall of the vessel 180. FIG. 10 shows a cross-section of the vessel 180 with the imaging element 90 of the inner member 70 disposed between the hook ends 125 of filter legs 105 engaged with the wall of the vessel 180. After visual confirmation of the implanted filter 95, the inner member 70 is retracted back into the center lumen 115 of the elongate body 25, and the delivery device 100 can be removed from the vessel lumen 185 (as shown in FIG. 11).

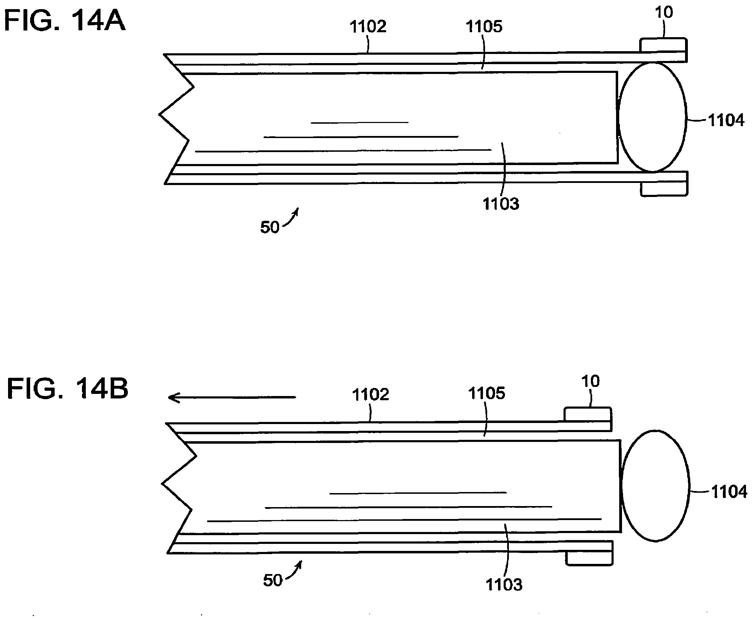

FIGS. 14A and 14B illustrate another embodiment of the delivery device 100. In this embodiment, the catheter is deployed by translating an outer sheath with respect to an inner sheath. The distal portion 50 of the delivery device 100according to this embodiment is depicted in FIGS. 14A and 14B. The delivery device 100 includes an outer sheath 1102 and an inner sheath 1103 disposed within a lumen 1105 of the outer sheath 1102. The outer sheath may include an imaging element 10 at the distal end. The imaging element 10 may have the same configuration on the outer sheath 1102 as it does on the elongate body 25 of the embodiment depicted in FIGS. 1-3. In the un-deployed state (FIG. 14A), an implant 1104 (such as implant 95, 200, 204) is disposed within the lumen 1105 of the outer sheath 1102 and rests against the distal end of the inner sheath 1102. For deployment, the outer sheath 1102 translates proximally with respect to the inner sheath 110 as shown in FIG. 14B. Due to the proximal translation of the outer sheath 1102, the implant 1104 is deployed into the vessel.

Catheter bodies intended for intravascular introduction, such as the elongate body of the delivery device, will typically have a length in the range from 50 cm to 200 cm and an outer diameter in the range from 1 French to 12 French (0.33 mm: 1 French), usually from 3 French to 9 French. In the case of coronary catheters, the length is typically in the range from 125 cm to 200 cm, the diameter is preferably below 8 French, more preferably below 7 French, and most preferably in the range from 2 French to 7 French. Catheter bodies will typically be composed of an organic polymer that is fabricated by conventional extrusion techniques. Suitable polymers include polyvinylchloride, polyurethanes, polyesters, polytetrafluoroethylenes (PTFE), silicone rubbers, natural rubbers, and the like. Optionally, the catheter body may be reinforced with braid, helical wires, coils, axial filaments, or the like, in order to increase rotational strength, column strength, toughness, pushability, and the like. Suitable catheter bodies may be formed by extrusion, with one or more channels being provided when desired. The catheter diameter can be modified by heat expansion and shrinkage using conventional techniques. The resulting catheters will thus be suitable for introduction to the vascular system, often the coronary arteries, by conventional techniques.

The distal portion of the catheters of the present invention may have a wide variety of forms and structures. In many embodiments, a distal portion of the catheter is more rigid than a proximal portion, but in other embodiments the distal portion may be equally as flexible as the proximal portion. One aspect of the present invention provides catheters having a distal portion with a reduced rigid length. The reduced rigid length can allow the catheters to access and treat tortuous vessels and small diameter body lumens. In most embodiments a rigid distal portion or housing of the catheter body will have a diameter that generally matches the proximal portion of the catheter body, however, in other embodiments, the distal portion may be larger or smaller than the flexible portion of the catheter.

A rigid distal portion of a catheter body can be formed from materials that are rigid or which have very low flexibilities, such as metals, hard plastics, composite materials, NiTi, steel with a coating such as titanium nitride, tantalum, ME-92 (antibacterial coating material), diamonds, or the like. Most usually, the distal end of the catheter body will be formed from stainless steel or platinum/iridium. The length of the rigid distal portion may vary widely, typically being in the range from 5 mm to 35 mm, more usually from 10 mm to 25 mm, and preferably between 6 mm and 8 mm. In contrast, conventional catheters typically have rigid lengths of approximately 16 mm. The opening 1001 of the present invention will typically have a length of approximately 2 mm. In other embodiments, however, the opening can be larger or smaller.

The inner member disposed within the delivery system can include any suitable material having a shaft with enough rigidity to deploy an implant while being flexible enough to move through a body lumen. Like the catheter, the inner member can be formed from polymers optionally reinforced with braid, helical wires, coils, axial filaments, or the like, in order to increase rotational strength, column strength, toughness, pushability, and the like. Suitable polymers include polyvinylchloride, polyurethanes, polyesters, polytetrafluoroethylenes (PTFE), silicone rubbers, natural rubbers, and the like.

According to certain embodiments, the delivery device includes one or more imaging elements. In certain aspects, the elongate body of the delivery device includes an imaging element and the inner member of the delivery device includes an imaging element. The imaging element of the elongate body and the imaging element of the inner member may be the same or different. Imaging elements suitable for use with the delivery devices of the invention are described hereinafter. The imaging element is a component of an imaging assembly. Any imaging assembly may be used with devices of the invention, such as optical-acoustic imaging apparatus, intravascular ultrasound (IVUS) or optical coherence tomography (OCT). The imaging element is used to send and receive signals to and from the imaging surface that form the imaging data.

The imaging assembly may be an intravascular ultrasound (IVUS) imaging assembly. IVUS uses an ultrasound probe attached at the distal end. The ultrasound probe can either be either a rotating transducer or an array of circumferentially positioned transducers. For example and as shown in throughout the figures (e.g. FIG. 3), the ultrasound probe can be the imaging element 10 on the elongate body 25 and/or imaging element 90 on the inner member 70. The proximal end of the catheter is attached to computerized ultrasound equipment. The IVUS imaging element (i.e. ultrasound probe) includes transducers that image the tissue with ultrasound energy (e.g., 20-50 MHz range) and image collectors that collect the returned energy (echo) to create an intravascular image. The imaging transducers and imaging collectors are coupled to signal lines that run through the length of the catheter and couple to the computerized ultrasound equipment. For example, the signal lines 65 and 85 coupled to the imaging elements shown throughout the Figures, including in FIG. 3.

IVUS imaging assemblies produce ultrasound energy and receive echoes from which real time ultrasound images of a thin section of the blood vessel are produced. The imaging transducers of the imaging element are constructed from piezoelectric components that produce sound energy at 20-50 MHz. The image collectors of the imaging element comprise separate piezoelectric elements that receive the ultrasound energy that is reflected from the vasculature. Alternative embodiments of imaging assembly may use the same piezoelectric components to produce and receive the ultrasonic energy, for example, by using pulsed ultrasound. That is, the imaging transducer and the imaging collectors are the same. Another alternative embodiment may incorporate ultrasound absorbing materials and ultrasound lenses to increase signal to noise.

IVUS data is typically gathered in segments where each segment represents an angular portion of an IVUS image. Thus, it takes a plurality of segments (or a set of IVUS data) to image an entire cross-section of a vascular object. Furthermore, multiple sets of IVUS data are typically gathered from multiple locations within a vascular object (e.g., by moving the transducer linearly through the vessel). These multiple sets of data can then be used to create a plurality of two-dimensional (2D) images or one three-dimensional (3D) image.

IVUS imaging assemblies and processing of IVUS data are described in further detail in, for example, Yock, U.S. Pat. Nos. 4,794,931, 5,000,185, and 5,313,949; Sieben et al., U.S. Pat. Nos. 5,243,988, and 5,353,798; Crowley et al., U.S. Pat. No. 4,951,677; Pomeranz, U.S. Pat. No. 5,095,911, Griffith et al., U.S. Pat. No. 4,841,977, Maroney et al., U.S. Pat. No. 5,373,849, Born et al., U.S. Pat. No. 5,176,141, Lancee et al., U.S. Pat. No. 5,240,003, Lancee et al., U.S. Pat. No. 5,375,602, Gardineer et at., U.S. Pat. No. 5,373,845, Seward et al., Mayo Clinic Proceedings 71(7):629-635 (1996), Packer et al., Cardiostim Conference 833 (1994), "Ultrasound Cardioscopy," Eur. J.C.P.E. 4(2):193 (June 1994), Eberle et al., U.S. Pat. No. 5,453,575, Eberle et al., U.S. Pat. No. 5,368,037, Eberle et at., U.S. Pat. No. 5,183,048, Eberle et al., U.S. Pat. No. 5,167,233, Eberle et at., U.S. Pat. No. 4,917,097, Eberle et at., U.S. Pat. No. 5,135,486, U.S. Pub. 2009/0284332; U.S. Pub. 2009/0195514 A1; U.S. Pub. 2007/0232933; and U.S. Pub. 2005/0249391 and other references well known in the art relating to intraluminal ultrasound devices and modalities.

In other embodiments, the imaging assembly may be an optical coherence tomography imaging assembly. OCT is a medical imaging methodology using a miniaturized near infrared light-emitting probe. As an optical signal acquisition and processing method, it captures micrometer-resolution, three-dimensional images from within optical scattering media (e.g., biological tissue). Recently it has also begun to be used in interventional cardiology to help diagnose coronary artery disease. OCT allows the application of interferometric technology to see from inside, for example, blood vessels, visualizing the endothelium (inner wall) of blood vessels in living individuals.

OCT systems and methods are generally described in Castella et al., U.S. Patent No. 8,108,030, Milner et al., U.S. Patent Application Publication No. 2011/0152771, Condit et al., U.S. Patent Application Publication No. 2010/0220334, Castella et al., U.S. Patent Application Publication No. 2009/0043191, Milner et al., U.S. Patent Application Publication No. 2008/0291463, and Kemp, N., U.S. Patent Application Publication No. 2008/0180683. In OCT, a light source delivers a beam of light to an imaging device to image target tissue. Light sources can include pulsating light sources or lasers, continuous wave light sources or lasers, tunable lasers, broadband light source, or multiple tunable laser. Within the light source is an optical amplifier and a tunable filter that allows a user to select a wavelength of light to be amplified. Wavelengths commonly used in medical applications include near-infrared light, for example between about 800 nm and about 1700 nm.

Aspects of the invention may obtain imaging data from an OCT system, including OCT systems that operate in either the time domain or frequency (high definition) domain. Basic differences between time-domain OCT and frequency-domain OCT is that in time-domain OCT, the scanning mechanism is a movable mirror, which is scanned as a function of time during the image acquisition. However, in the frequency-domain OCT, there are no moving parts and the image is scanned as a function of frequency or wavelength.

In time-domain OCT systems an interference spectrum is obtained by moving the scanning mechanism, such as a reference mirror, longitudinally to change the reference path and match multiple optical paths due to reflections within the sample. The signal giving the reflectivity is sampled over time, and light traveling at a specific distance creates interference in the detector. Moving the scanning mechanism laterally (or rotationally) across the sample produces two-dimensional and three-dimensional images.

In frequency domain OCT, a light source capable of emitting a range of optical frequencies excites an interferometer, the interferometer combines the light returned from a sample with a reference beam of light from the same source, and the intensity of the combined light is recorded as a function of optical frequency to form an interference spectrum. A Fourier transform of the interference spectrum provides the reflectance distribution along the depth within the sample.

Several methods of frequency domain OCT are described in the literature. In spectral-domain OCT (SD-OCT), also sometimes called "Spectral Radar" (Optics letters, Vol. 21, No. 14 (1996) 1087-1089), a grating or prism or other means is used to disperse the output of the interferometer into its optical frequency components. The intensities of these separated components are measured using an array of optical detectors, each detector receiving an optical frequency or a fractional range of optical frequencies. The set of measurements from these optical detectors forms an interference spectrum (Smith, L. M. and C. C. Dobson, Applied Optics 28: 3339-3342), wherein the distance to a scatterer is determined by the wavelength dependent fringe spacing within the power spectrum. SD-OCT has enabled the determination of distance and scattering intensity of multiple scatters lying along the illumination axis by analyzing a single the exposure of an array of optical detectors so that no scanning in depth is necessary. Typically the light source emits a broad range of optical frequencies simultaneously.

Alternatively, in swept-source OCT, the interference spectrum is recorded by using a source with adjustable optical frequency, with the optical frequency of the source swept through a range of optical frequencies, and recording the interfered light intensity as a function of time during the sweep. An example of swept-source OCT is described in U.S. Pat. No. 5,321,501.

Generally, time domain systems and frequency domain systems can further vary in type based upon the optical layout of the systems: common beam path systems and differential beam path systems. A common beam path system sends all produced light through a single optical fiber to generate a reference signal and a sample signal whereas a differential beam path system splits the produced light such that a portion of the light is directed to the sample and the other portion is directed to a reference surface. Common beam path systems are described in U.S. Pat. 7,999,938; U.S. Pat. 7,995,210; and U.S. Pat. 7,787,127 and differential beam path systems are described in U.S. Pat. 7,783,337; U.S. Pat. 6,134,003; and U.S. Pat. 6,421,164. In yet another embodiment, the imaging assembly is an optical-acoustic imaging apparatus. Optical-acoustic imaging apparatus include at least one imaging element to send and receive imaging signals. In one embodiment, the imaging element includes at least one acoustic-to-optical transducer. In certain embodiments, the acoustic-to-optical transducer is an Fiber Bragg Grating within an optical fiber. In addition, the imaging elements may include the optical fiber with one or more Fiber Bragg Gratings (acoustic-to-optical transducer) and one or more other transducers. The at least one other transducer may be used to generate the acoustic energy for imaging. Acoustic generating transducers can be electric-to-acoustic transducers or optical-to-acoustic transducers. The imaging elements suitable for use in devices of the invention are described in more detail below.

Fiber Bragg Gratings for imaging provides a means for measuring the interference between two paths taken by an optical beam. A partially-reflecting Fiber Bragg Grating is used to split the incident beam of light into two parts, in which one part of the beam travels along a path that is kept constant (constant path) and another part travels a path for detecting a change (change path). The paths are then combined to detect any interferences in the beam. If the paths are identical, then the two paths combine to form the original beam. If the paths are different, then the two parts will add or subtract from each other and form an interference. The Fiber Bragg Grating elements are thus able to sense a change wavelength between the constant path and the change path based on received ultrasound or acoustic energy. The detected optical signal interferences can be used to generate an image using any conventional means.

Exemplary optical-acoustic imaging assemblies are disclosed in more detail in U.S. Patent Nos. 6,659,957 and 7,527,594, 7,245.789, 7447,388, 7,660,492, 8,059,923 and in U.S. Patent Publication Nos. 2008/0119739, 2010/0087732 and 2012/0108943.

In some embodiments, a device of the invention includes an imaging assembly and obtains a three-dimensional data set through the operation of OCT, IVUS, or other imaging hardware. In some embodiments, a device of the invention is a computer device such as a laptop, desktop, or tablet computer, and obtains a three-dimensional data set by retrieving it from a tangible storage medium, such as a disk drive on a server using a network or as an email attachment.

Methods of the present disclosure can be performed using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections).

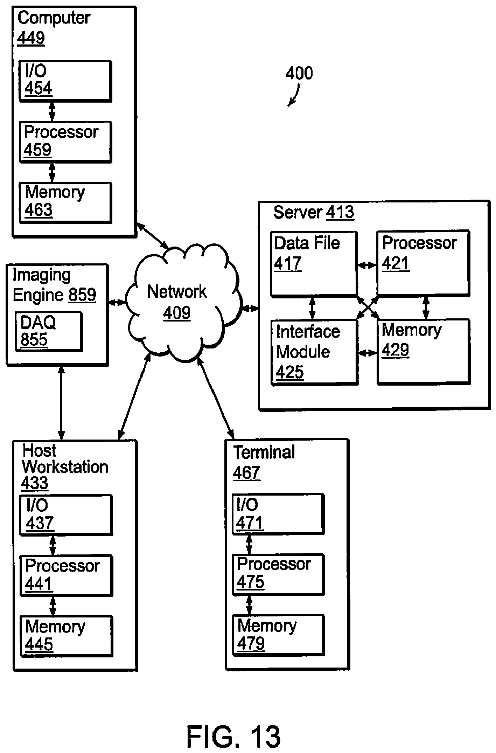

In some embodiments, a user interacts with a visual interface to view images from the imaging system. Input from a user (e.g., parameters or a selection) are received by a processor in an electronic device. The selection can be rendered into a visible display. An exemplary system including an electronic device is illustrated in FIG. 13. As shown in FIG. 13, an imaging engine 859 of the imaging assembly communicates with host workstation 433 as well as optionally server 413 over network 409. The data acquisition element 855 (DAQ) of the imaging engine receives imaging data from one or more imaging element. In some embodiments, an operator uses computer 449 or terminal 467 to control system 400 or to receive images. An image may be displayed using an I/O 454, 437, or 471, which may include a monitor. Any I/O may include a keyboard, mouse or touchscreen to communicate with any of processor 421, 459, 441, or 475, for example, to cause data to be stored in any tangible, nontransitory memory 463, 445, 479, or 429. Server 413 generally includes an interface module 425 to effectuate communication over network 409 or write data to data file 417.

Processors suitable for the execution of computer program include, by way of example, both general and special purpose microprocessors, and any one or more processor of any kind of digital computer. Generally, a processor will receive instructions and data from a read-only memory or a random access memory or both. The essential elements of computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, solid state drive (SSD), and flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can be implemented on a computer having an I/O device, e.g., a CRT, LCD, LED, or projection device for displaying information to the user and an input or output device such as a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The subject matter described herein can be implemented in a computing system that includes a back-end component (e.g., a data server 413), a middleware component (e.g., an application server), or a front-end component (e.g., a client computer 449 having a graphical user interface 454 or a web browser through which a user can interact with an implementation of the subject matter described herein), or any combination of such back-end, middleware, and front-end components. The components of the system can be interconnected through network 409 by any form or medium of digital data communication, e.g., a communication network. Examples of communication networks include cell network (e.g., 3G or 4G), a local area network (LAN), and a wide area network (WAN), e.g., the Internet.

The subject matter described herein can be implemented as one or more computer program products, such as one or more computer programs tangibly embodied in an information carrier (e.g., in a non-transitory computer-readable medium) for execution by, or to control the operation of, data processing apparatus (e.g., a programmable processor, a computer, or multiple computers). A computer program (also known as a program, software, software application, app, macro, or code) can be written in any form of programming language, including compiled or interpreted languages (e.g., C, C++, Perl), and it can be deployed in any form, including as a stand-alone program or as a module, component, subroutine, or other unit suitable for use in a computing environment. Systems and methods of the invention can include instructions written in any suitable programming language known in the art, including, without limitation, C, C++, Perl, Java, ActiveX, HTML5, Visual Basic, or JavaScript.

A computer program does not necessarily correspond to a file. A program can be stored in a portion of file 417 that holds other programs or data, in a single file dedicated to the program in question, or in multiple coordinated files (e.g., files that store one or more modules, subprograms, or portions of code). A computer program can be deployed to be executed on one computer or on multiple computers at one site or distributed across multiple sites and interconnected by a communication network.

A file can be a digital file, for example, stored on a hard drive, SSD, CD, or other tangible, non-transitory medium. A file can be sent from one device to another over network 409 (e.g., as packets being sent from a server to a client, for example, through a Network Interface Card, modem, wireless card, or similar).

Writing a file according to the invention involves transforming a tangible, non-transitory computer-readable medium, for example, by adding, removing, or rearranging particles (e.g., with a net charge or dipole moment into patterns of magnetization by read/write heads), the patterns then representing new collocations of information about objective physical phenomena desired by, and useful to, the user. In some embodiments, writing involves a physical transformation of material in tangible, non-transitory computer readable media (e.g., with certain optical properties so that optical read/write devices can then read the new and useful collocation of information, e.g., burning a CD-ROM). In some embodiments, writing a file includes transforming a physical flash memory apparatus such as NAND flash memory device and storing information by transforming physical elements in an array of memory cells made from floating-gate transistors. Methods of writing a file are well-known in the art and, for example, can be invoked manually or automatically by a program or by a save command from software or a write command from a programming language.

## Claims

1. An implant delivery device (50, 100) comprising:
an elongate body (25) comprising a first imaging element (10) and defining a center lumen (115) that leads to an opening (20), wherein the elongate body is configured to releasably hold an implant (95) within the center lumen and the first imaging element is configured to at least partially surround the center lumen such that the implant is deployable through the first imaging element;
an inner member (70) being moveably disposable within the center lumen of the elongate body, wherein the inner member configured to engage with and deploy the implant out of the opening and into a body lumen;
wherein the inner member comprises a push element (120) and the push element is configured to engage with the implant, and
wherein the inner member comprises a second imaging element (90) located proximal to the push element of the inner member.

2. The implant delivery device of claim 1, wherein the second imaging element is configured to obtain images of the luminal surface when at least a portion of the inner member is deployed out of the opening.

3. The implant delivery device of any preceding claim, wherein the push element forms a distal end of the inner member, and the push element is configured to engage with the implant, as the inner member translates relative to the elongate body, to deploy the implant.

4. The implant delivery device of any preceding claim, wherein the first imaging element is selected from the group consisting of: an ultrasound assembly and an optical coherence tomography assembly.

5. The implant delivery device of any preceding claim, wherein the second imaging element is selected from the group consisting of: an ultrasound assembly and an optical coherence tomography assembly.

6. The implant delivery device of any preceding claim, wherein the first and second imaging elements are the same.

7. The implant delivery device of any preceding claim, wherein the implant comprises a filter that is expandable from a collapsed state, when the filter is held within the elongate body, to an expanded state, when the filter is deployed into the body lumen.

8. The implant delivery device of claim 7, wherein the filter is bioabsorbable.

## Patentansprüche

1. Implantatzuführungsvorrichtung (50, 100), die Folgendes umfasst:
einen länglichen Körper (25), der ein erstes bildgebendes Element (10) umfasst und ein mittleres Lumen (115) definiert, das zu einer Öffnung (20) führt, wobei der längliche Körper dazu ausgelegt ist, ein Implantat (95) lösbar im mittleren Lumen zu halten, und das erste bildgebende Element dazu ausgelegt ist, das mittlere Lumen mindestens teilweise derart zu umgeben, dass das Implantat durch das erste bildgebende Element entfaltbar ist;
eine innere Komponente (70), die im mittleren Lumen des länglichen Körpers bewegbar anordenbar ist, wobei die innere Komponente dazu ausgelegt ist, in das Implantat einzugreifen und dieses aus der Öffnung und in ein Körperlumen zu entfalten;
wobei die innere Komponente ein Druckelement (120) umfasst und das Druckelement dazu ausgelegt ist, in das Implantat einzugreifen, und
wobei die innere Komponente ein zweites bildgebendes Element (90) umfasst, das proximal zum Druckelement der inneren Komponente platziert ist.

2. Implantatzuführungsvorrichtung nach Anspruch 1, wobei das zweite bildgebende Element dazu ausgelegt ist, Bilder von der luminalen Oberfläche zu erhalten, wenn mindestens ein Abschnitt der inneren Komponente aus der Öffnung entfaltet ist.

3. Implantatzuführungsvorrichtung nach einem der vorhergehenden Ansprüche, wobei das Druckelement ein distales Ende der inneren Komponente bildet und das Druckelement dazu ausgelegt ist, in das Implantat einzugreifen, wenn die innere Komponente bezogen auf den länglichen Körper umgesetzt wird, um das Implantat zu entfalten.

4. Implantatzuführungsvorrichtung nach einem der vorhergehenden Ansprüche, wobei das erste bildgebende Element aus der Gruppe ausgewählt wird, die besteht aus: einer Ultraschallanordnung und einer optischen Kohärenztomographieanordnung.

5. Implantatzuführungsvorrichtung nach einem der vorhergehenden Ansprüche, wobei das zweite bildgebende Element aus der Gruppe ausgewählt wird, die besteht aus: einer Ultraschallanordnung und einer optischen Kohärenztomographieanordnung.

6. Implantatzuführungsvorrichtung nach einem der vorhergehenden Ansprüche, wobei das erste und das zweite bildgebende Element dasselbe sind.

7. Implantatzuführungsvorrichtung nach einem der vorhergehenden Ansprüche, wobei das Implantat einen Filter umfasst, der aus einem eingeklappten Zustand, wenn der Filter im länglichen Körper gehalten wird, in einen ausgeklappten Zustand, wenn der Filter in das Körperlumen entfaltet wird, ausklappbar ist.

8. Implantatzuführungsvorrichtung nach Anspruch 7, wobei der Filter bioabsorbierbar ist.

## Revendications

1. Dispositif de pose d'implant (50, 100) comprenant :
un corps allongé (25) comprenant un premier élément d'imagerie (10) et définissant une lumière centrale (115) qui conduit jusqu'à une ouverture (20), dans lequel le corps allongé est conçu pour retenir de façon libérable un implant (95) à l'intérieur de la lumière centrale et le premier élément d'imagerie est conçu pour entourer au moins partiellement la lumière centrale de façon que l'implant soit déployable à travers le premier élément d'imagerie ;
un organe interne (70) pouvant être disposé de façon mobile à l'intérieur de la lumière centrale du corps allongé, dans lequel l'organe interne est conçu pour s'appliquer sur l'implant et le déployer hors de l'ouverture et dans une lumière corporelle ;
dans lequel l'organe interne comprend un élément de poussée (120) et l'élément de poussée est conçu pour venir au contact de l'implant, et
dans lequel l'organe interne comprend un second élément d'imagerie (90) situé à proximité de l'élément de poussée de l'organe interne.

2. Dispositif de pose d'implant selon la revendication 1, dans lequel le second élément d'imagerie est conçu pour obtenir des images de la surface luminale quand au moins une partie de l'organe interne est déployée hors de l'ouverture.

3. Dispositif de pose d'implant selon une quelconque revendication précédente, dans lequel l'élément de poussée forme une extrémité distale de l'organe interne, et l'élément de poussée est conçu pour venir au contact de l'implant, quand l'organe interne se déplace par rapport au corps allongé, pour déployer l'implant.

4. Dispositif de pose d'implant selon une quelconque revendication précédente, dans lequel le premier élément d'imagerie est choisi dans le groupe constitué : d'un ensemble ultrasonore et d'un ensemble tomographie en cohérence optique.

5. Dispositif de pose d'implant selon une quelconque revendication précédente, dans lequel le second élément d'imagerie est choisi dans le groupe constitué : d'un ensemble ultrasonore et d'un ensemble tomographie en cohérence optique.

6. Dispositif de pose d'implant selon une quelconque revendication précédente, dans lequel les premier et second éléments d'imagerie sont identiques.

7. Dispositif de pose d'implant selon une quelconque revendication précédente, dans lequel l'implant comprend un filtre qui peut être agrandi à partir d'un état rétracté, quand le filtre est maintenu à l'intérieur du corps allongé, vers un état agrandi, quand le filtre est déployé dans la lumière corporelle.

8. Dispositif de pose d'implant selon la revendication 7, dans lequel le filtre est bioabsorbable.
